(19) Europäisches Patentamt
European Patent Office
Office européen des brevets

(11) Numéro de publication: **0 015 214**
**B1**

# (12) FASCICULE DE BREVET EUROPEEN

(45) Date de publication du fascicule de brevet: **27.01.82**

(21) Numéro de dépôt: **80400261.6**

(22) Date de dépôt: **25.02.80**

(51) Int. Cl.³: **C 07 C 103/50,** C 07 C 121/78, C 07 C 149/42, C 07 D 295/14, A 61 K 31/16, A 61 K 31/395

(54) **Nouveaux dérivés de benzoyl-2 glycylanilides substitués, leur préparation et leur application en tant que médicaments anxiolytiques.**

(30) Priorité: **26.02.79 FR 7904837**

(43) Date de publication de la demande: ***03.09.80 Bulletin 80/18***

(45) Mention de la délivrance du brevet: **27.01.82 Bulletin 82/4**

(84) Etats contractants désignés: ***AT BE CH DE GB IT LU NL SE***

(56) Documents cités:
**EP-A-0 000 299**
**FR-A-2 244 482**
**US-A-3 455 985**

(73) Titulaire: **PIERRE FABRE S.A., 125, rue de la Faisanderie, F-75116 Paris (FR)**

(72) Inventeur: ***Mouzin, Gilbert, 21, rue Sainte Foy,* F-81100 Castres (FR)**
Inventeur: **Cousse, Henri, Chemin de Lastinos, F-81100 Castres (FR)**

(74) Mandataire: **Corre, Jacques Denis Paul, Cabinet Regimbeau 26, Avenue Kléber, F-75116 Paris (FR)**

EP 0 015 214 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveaux dérivés de benzoyl-2 glycylanilides substitués, leur préparation et leur application en tant que médicaments anxiolytiques

La présente invention réalisée au Centre de Recherches Pierre Fabre concerne de nouveaux dérivés de glycylanilides substitués, leur procédé de préparation et leur application en tant que médicament.

Dans la technique antérieure se trouvent déjà décrits des composés de formule générale

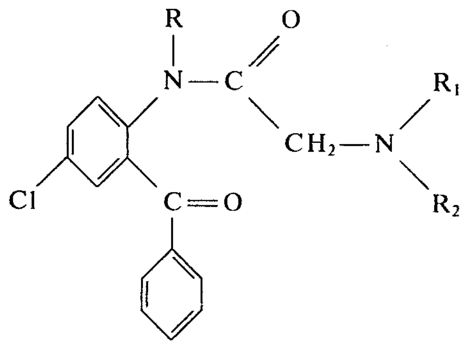

présentés comme pouvant être utilisés en thérapeutique dans le traitement de l'anxiété et de névrose. Une telle technique antérieure peut par exemple être illustrée par la demande de brevet européen n° 78400009.3 et par le brevet américain n° 3 455 985.

Par rapport à ces composés de la technique antérieure la présente demande propose de nouveaux dérivés dotés d'une activité différente du fait de l'absence de leur composante anti-convulsivante ou du fait d'une activité pharmacologique nouvelle, à savoir de leur activité hypnotique.

Les nouveaux dérivés de l'invention répondent à la formule générale I

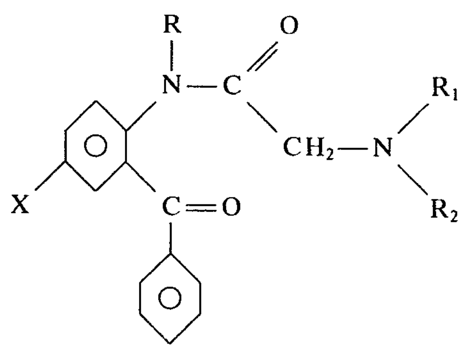

(I)

dans laquelle:

X représente un atome d'hydrogène, de brome, de fluor ou d'iode, ou un groupe alcoyle inférieur, alcoxy inférieur, un groupe cyané, un groupe trifluorométhyle, un groupe alcoyl-mercapto ou un groupe dialcoylamino;

R représente un groupe alcoyle inférieur;

$R_1$ et $R_2$ peuvent être identiques ou différents et sont choisis parmi l'hydrogène, les groupes alcoyle inférieur, hydroxy-alcoyle inférieur, alcényle inférieur, alcynyle inférieur, un cycloalcoyle de 3 à 6 chaînons éventuellement substitués par un radical alcoyle inférieur; ils peuvent en outre former avec l'atome d'azote auquel ils sont reliés, un hétérocycle azoté comprenant éventuellement un second hétéroatome choisi parmi l'oxygène et l'azote;

à la condition que lorsque $R_1 = H$, $R_2$ est différent d'un alcoyle inférieur ou d'un groupe hydroxyalcoyle inférieur, et

$R_1$ et $R_2$ ne peuvent représenter simultanément un atome d'hydrogène ou un groupe alcoyle inférieur.

On remarquera qu'en aucun cas X ne peut être un atome de chlore.

On notera que les termes alcoyle inférieur, alcényle inférieur et alcynyle inférieur désignent des groupes contenant de 1 à 4 atomes de carbone.

L'invention s'applique également aux sels des composés de formule (I) obtenus avec des acides thérapeutiquement acceptables.

A titre d'exemples non limitatifs de sels d'addition thèrapeutiquement ou physiologiquement acceptables, on peut citer les sels d'acides minéraux, tels que les acides chlorhydrique, phosphorique et sulfurique et les sels d'acides organiques, tels que les acides succinique, maléique, fumarique, citrique, etc.

La présente invention concerne également un procédé de préparation des composés de formule I caractérisé en ce que l'on condense un composé de formule II:

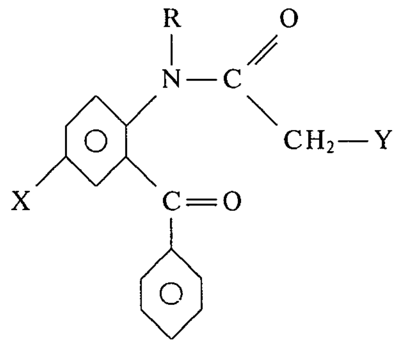

(II)

dans laquelle:

X et R ont les significations données à propos de la formule générale I, et
Y représente un atome d'halogène

avec une amine de formule générale III

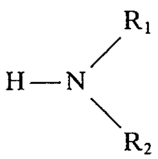

(III)

dans laquelle:

$R_1$ et $R_2$ ont les significations données à propos de la formule générale I.

Les intermédiaires de synthèse de formule II peuvent être préparés à partir d'amino benzophénones obtenues:

1°) Par condensation d'anilines substituées, avec du chlorure de benzoyle en utilisant du chlorure de zinc comme catalyseur, puis hydrolyse du produit de la réaction (L. H. Sternbach, R. I. Freyer, W. Metlesics, G. Sach et A. Stempel — J. Org. Chem. 1962, 27, 3781):

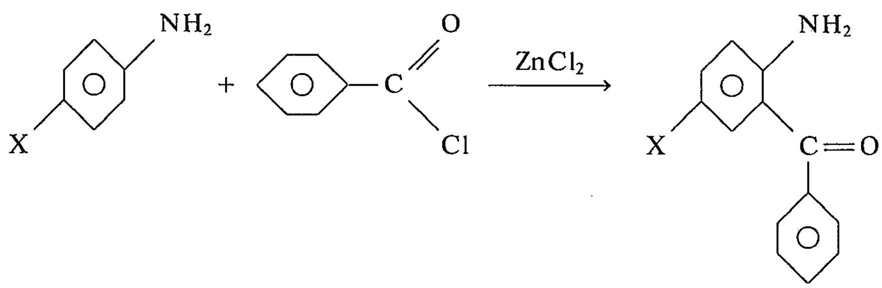


2°) Par l'intermédiaire d'une synthèse indolique (L. H. Sternbach — J. Org. Chem. 1962, 27, 3781):

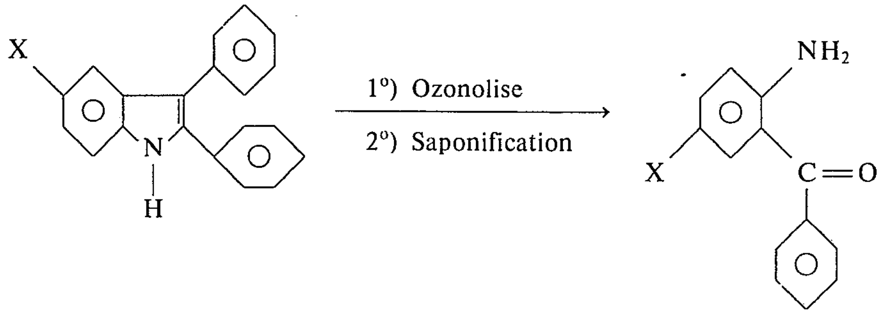

3°) A partir de 2-1 benzisoxazoles (R. Y. Ning, P. B. Madan, L. H. Sternbach — J. Heterocyclic 1974, 11, 107):

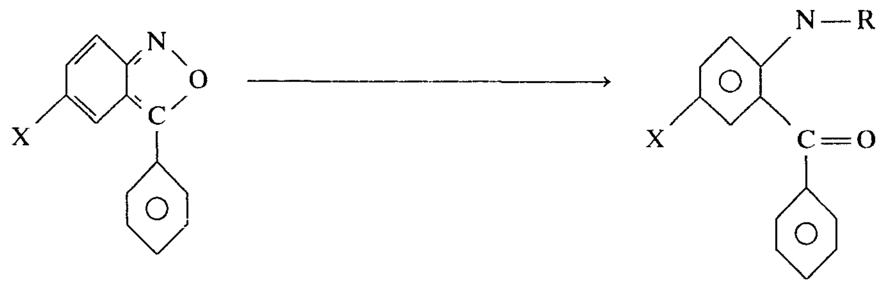

4°) Par la méthode de Lothrop et Goodwin — J. Am. Chem. Soc., 1943, 65, 363.
En traitant par un magnésien une benzoxazinone

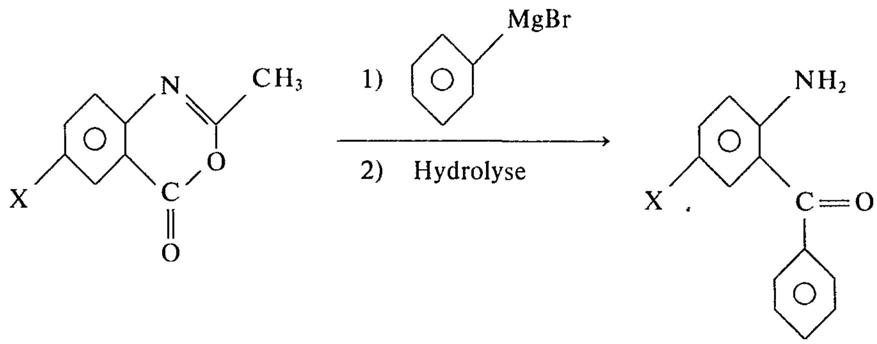


l'alcoylation sélective des amino benzophénones peut être effectuée par catalyse par transfert de phase, selon le procédé décrit par G. Mouzin et H. Cousse (dans le brevet français n° 78.26918).

Les diverses amino benzophénones sont ensuite traitées selon le schéma réactionnel:

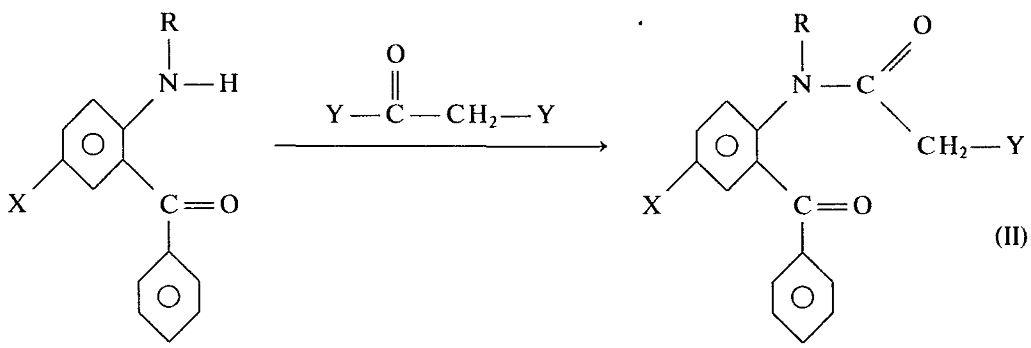

(II)

L'invention concerne enfin l'application des composés de formule générale I en tant que médicaments doués d'une activité sur le système nerveux central et, en particulier, en tant qu'agents anxiolytiques, sédatifs, anti-convulsifs, hypnotiques ou comme agent de relaxation musculaire.

La présente invention sera décrite ci-après plus en détails à propos des exemples non limitatifs suivants:

## Exemple 1

N-méthyl benzoyl-2′ morpholino-2 acétanilide

### (a) Préparation de la tosylamino-2 benzophénone

A une solution de 19,72 g (0,1 mole) d'amino benzophénone dans 250 cm³ de pyridine, on ajoute par spatulées 22,87 g (0,12 mole) de chlorure de tosyle.

On chauffe à 100° C pendant 4 heures, puis on laisse revenir à la température ambiante et l'on jette sur 1500 cm³ d'eau glacée contenant 200 cm³ d'acide chlorhydrique.

On extrait à l'acétate d'éthyle, on lave à l'eau jusqu'à neutralité et l'on sèche. On évapore jusqu'à siccité et l'on reprend le résidu cristallin dans l'éther de pétrole.

On récupère 30,43 g de cristaux. Rendement 87%. Point de fusion: 128° C.
Chromatographie sur plaque:

- support: gel de silice 60 F 254 Merck
- solvant: acétate d'èthyle — èther de pétrole 10/90
- révélation: U. V. et iode
- Rf: 0,82

(b) N-méthyl benzoyl-2' tosanilide

Dans une solution de 570 $cm^3$ de toluène, on introduit 40 g de tosylamino benzophénone (0,114 mole), 120 $cm^3$ de lessive de soude et 800 mg de chlorure de benzyltriéthyl ammonium. La suspension obtenue est agitée pendant 20 minutes, puis on additionne 24,3 $cm^3$ (0,256 mole) de sulfate de méthyle. On maintient pendant 1 heure sous forte agitation, puis on introduit un mélange eau — acétate d'éthyle et l'on décante.

On lave la phase organique jusqu'à neutralité, sèche sur sulfate de sodium, décolore au noir animal, on filtre et l'on évapore jusqu'à siccité.

Le résidu obtenu est recristallisé dans un mélange hexane — acétate d'éthyle.

On récupère 45 g de cristaux. Rendement quantitatif. Point de fusion: 129° C.
Chromatographie sur plaque:

- support: gel de silice 60 F 254 Merck
- solvant: acétate d'éthyle — éther de pétrole 30/70
- révélation: U.V. et iode
- Rf: 0,57

(c) Méthyl amino benzophénone

On prépare une solution d'acide sulfurique à 70% par addition lente de 245 $cm^3$ d'acide sulfurique dans 110 g de glace pilée. A cette solution portée à 100° C, on ajoute 45 g de N-méthyl benzoyl-2' tosanilide (0,144 mole). On laisse pendant 20 minutes à cette température, puis après refroidissement, on jette sur 1 kg de glace pilée.

On neutralise par du bicarbonate de sodium et l'on extrait par l'acétate d'éthyle. On lave à l'eau, sèche sur sulfate de sodium, filtre et on évapore jusqu'à siccité.

Le résidu cristallin est recristallisé dans l'éther de pétrole. On obtient 23,1 g de cristaux jaunes. Rendement: 95%. Point de fusion: 68° C.
Chromatographie sur plaque:

- support: gel de silice 60 F 254 Merck
- solvant: acétate d'éthyle — éther de pétrole 30/70
- révélation: U.V. et iode
- Rf: 0,89

(d) N-méthyl benzoyl-2' bromo-2 acétanilide

A une solution jaune de 10,56 g (0,05 mole) de méthylaminobenzophénone dans 50 $cm^3$ d'acétate d'éthyle, on ajoute quelques morceaux de glace, puis on additionne lentement 4,85 $cm^3$ (0,055 mole) de bromure de bromacétyle.

On laisse revenir à température ambiante. Après 2 heures d'agitation, on extrait par de l'acétate d'éthyle. On lave à la soude (N) et à l'eau jusqu'à neutralité. On sèche sur sulfate de sodium, on filtre et on évapore sous vide jusqu'à siccité.

Le résidu est recristallisé dans l'acétate d'éthyle — éther de pétrole.

On récupère 15,5 g de cristaux jaunes (rendement 93%). Point de fusion: 97° C.
Chromatographie sur plaque:

- support: gel de silice 60 F 254 Merck
- solvant: acétate d'éthyle — éther de pétrole 30/70
- révélation: U.V. et iode
- Rf: 0,40

(e) N-méthyl benzoyl-2′ morpholino-2 acétanilide

A une solution de 6,49 g (0,0195 mole) de N-méthyl benzoyl-2′ bromo-2 acétanilide dans 65 $cm^3$ d'acétone, on ajoute rapidement 5 $cm^3$ (0,057 mole) de morpholine.

La solution se trouble, et des cristaux blancs se forment. On laisse une heure sous forte agitation, puis on évapore l'acétone. Le résidu est recristallisé dans l'hexane—acétate d'éthyle. On récupère avec un rendement de 85%, 5,64 g de produit de formule:

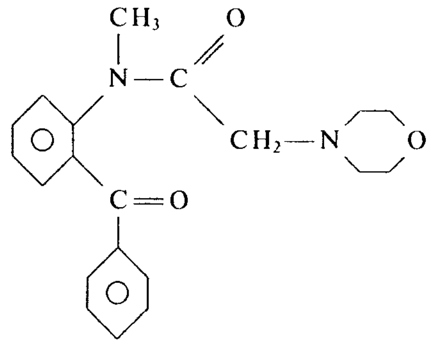

Formule brute: $C_{20}H_{22}N_2O_3$
Masse moléculaire: 338,41
Cristaux blancs
Point de fusion: 115° C
Chromatographie sur plaque:
- support: gel de silice 60 F 254 Merck
- solvant: butanol — acide acétique — eau 6/2/2
- révélation: U.V. et iode
- Rf: 0,45

Exemple 2

N-N′ diméthyl N(hydroxy-2 éthyl) benzoyl-2′ glycylanilide

Les quatre premières étapes sont identiques à celles mentionnées dans l'Exemple 1.

A une solution de 6,64 g de N′ méthyl benzoyl-2′ bromo-2 acétanilide (0,02 mole) en solution dans 50 $cm^3$ d'acétone, on ajoute 3 g (0,04 mole) de N-méthyl éthanolamine et l'on agite le mélange réactionnel pendant 20 heures à la température ambiante.

Après évaporation du solvant, on reprend par une solution de bicarbonate de sodium et l'on extrait par de l'acétate d'éthyle. On lave la phase organique à l'eau jusqu'à neutralité, on sèche sur sulfate de sodium, on filtre et on évapore jusqu'à siccité.

Le résidu obtenu est recristallisé dans l'acétate d'éthyle. On récupère avec un rendement de 85% un produit de formule:

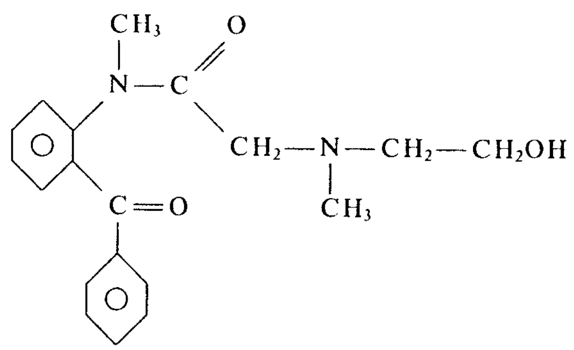

Formule brute: $C_{19}H_{22}N_2O_3$
Masse moléculaire: 326,39
Cristaux blancs
Point de fusion: 69° C
Chromatographie sur plaque:

- support: gel de silice 60 F 254 Merck
- solvant: méthanol – chloroforme 50/50
- révélation: U.V. et iode
- Rf: 0,65

Exemple 3

Maléate acide N-cyclohexyl N′ méthyl (benzoyl-2′ fluoro-4′) glycylanilide

(a) Préparation de l'amino-2 fluoro-5 benzophénone

A une solution de 46,44 g (0,4 mole) de chlorure de benzoyle chauffée à 120° C, on ajoute 18,9 cc (0,2 mole) de parafluoro aniline. On porte le mélange réactionnel à 180° C et on additionne par spatulées 32,7 g de chlorure de zinc, puis on chauffe pendant 2 heures à 220° C. On laisse revenir la masse réactionnelle à 140° C et on ajoute avec précaution 75 $cm^3$ d'acide acétique et 92,5 $cm^3$ d'acide sulfurique + 50 cc d'eau.

Aprés 4 heures d'agitation à 140° C, on jette sur de la glace pilée et on neutralise par addition de carbonate de sodium. On extrait avec de l'acétate d'éthyle, on lave à l'eau, sèche sur sulfate de sodium, filtre et on évapore le solvant jusqu'à siccité. Le résidu est recristallisé dans l'hexane; on récupère 16 g de cristaux jaunes. Rendement 74%.
Point de fusion 115° C.
Chromatographie sur plaque:

- support: gel de silice 60 F 254 Merck
- solvant: acétate d'éthyle – éther de pétrole
- révélation: U.V. et iode
- Rf: 0,66

(b) Préparation de la méthyl-2 fluoro-5 benzophénone

A une solution de 13 g (0,06 mole) d'amino-2 fluoro-5 benzophénone dans 100 $cm^3$ de THF, on ajoute 960 mg (0,003 mole) de bromure de tétrabutylammonium puis 8,18 g (0,20 mole) de soude pulvérisée et 30 cc (0,3 mole) de sulfate de méthyle. On chauffe le mélange réactionnel pendant 3 heures au reflux, puis on évapore le THF sous vide et l'on reprend le résidu à l'éther. On lave à l'eau jusqu'à neutralité, sèche sur sulfate de sodium, on filtre et on évapore le solvant; après recristallisation, on récupère avec un rendement de 87% de cristaux jaunes.
Point de fusion: 77° C.
Chromatographie sur plaque:

- support: gel de silice 60 F 254 Merck
- solvant: acétate d'éthyle – éther de pétrole 10/90
- révélation: U.V. et iode
- Rf: 0,43

(c) N-méthyl benzoyl-2′ fluoro-4′ bromo-2 acétanilide

On dissout 3,5 g (0,016 mole) de méthylamino-2 fluorobenzophénone dans 20 cc d'acétate d'éthyle, on ajoute quelques glaçons et on introduit goutte à goutte 1,8 cc (0,02 mole) de bromure de bromacétyle.

On agite pendant une heure, on dilue à l'éther, décante et on lave à la soude (N), puis à l'eau jusqu'à neutralité.

On sèche sur sulfate de sodium, on filtre et l'on chasse le solvant. L'huile récupérée est triturée dans l'éther de pétrole jusqu'à cristallisation.

On récupère 3,9 g de cristaux blancs. Rendement: 89%.
Point de fusion: 69° C.
Chromatographie sur plaque:

- support: gel de silice 60 F 254 Merck
- solvant: acétate d'éthyle – éther de pétrole 30/70
- révélation: U.V. et iode
- Rf: 0,58

(d) Maléate acide N-cyclohexyl N' méthyl (benzoyl-2' fluoro-4') glycylanilide

On agite pendant 2 heures à température ambiante une solution de 3,8 g (0,014 mole) de N-méthyl benzoyl-2' fluoro-4' bromo-2 acétanilide et 4,6 cc (0,04 mole) de cyclohexylamine dans 20 cc d'acétone. La solution se trouble, on évapore le solvant, on extrait à l'acétate d'éthyle, on lave à l'eau, on sèche sur sulfate de sodium, on filtre et on évapore le solvant; le résidu est recristallisé dans isopropanol — éther isopropylique.

On récupère avec un rendement de 75% un produit de formule:

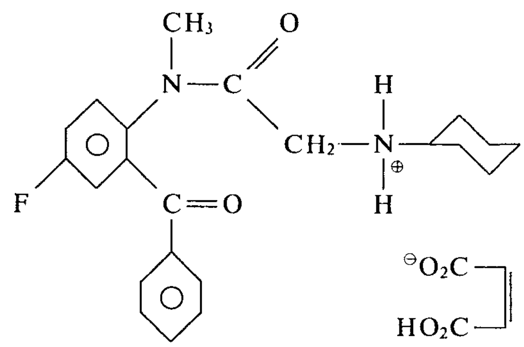

Formule brute: $C_{26}H_{29}FN_2O_6$
Masse moléculaire: 484,44
Cristaux blancs
Point de fusion: 144° C
Chromatographie sur plaque:
- support: gel de silice 60 F 254 Merck
- solvant: butanol — acide acétique — eau 6/2/2
- révélation: U.V. et iode
- Rf: 0,75

## Exemple 4

### Chlorhydrate de N-cyclohexyl N'-méthyl (benzoyl-2' bromo-4') glycylanilide

(a) Préparation de l'amino-2 bromo-5 benzophénone

A 116 cc (1 mole) de chlorure de benzoyle chauffés à 120°C, on ajoute 86 g (0,5 mole) de parabromo-aniline. On porte le mélange à 180° C et on additionne par spatulées 81,7 g (0,6 mole) de chlorure de zinc. On chauffe 2 heures à 220° C, puis on laisse revenir progressivement à 140° C.

On ajoute avec précaution 125 cc d'eau, 187 cc d'acide acétique et 230 cc d'acide sulfurique concentré.

On laisse 4 heures sous agitation à 140° C, puis on jette sur glace pilée; il se forme un précipité que l'on récupère par filtration. On reprend par de l'acétate d'éthyle, on lave à l'eau et à la soude (N) puis à l'eau jusqu'à neutralité.

On sèche sur sulfate de sodium, on filtre et on évapore sous pression réduite. Le résidu obtenu est recristallisé dans l'acétate d'éthyle — éther de pétrole.

On récupère 45 g de cristaux jaunes. Rendement: 70%.
Point de fusion: 112° C.
Chromatographie sur plaque:

- support: gel de silice 60 F 254 Merck
- solvant: acétate d'éthyle — éther de pétrole 30/70
- révélation: U.V. et iode
- Rf. 0,50.

(b) Préparation de la méthylamino-2 bromo-5 benzophénone

A une solution de 45 g (0,163 mole) d'amino-2 bromo-5 benzophénone dans 200 cm³ de THF, on ajoute 2,6 g (0,008 mole) de bromure de tétrabutylammonium, 22,2 g (0,55 mole) de soude pulvérisée et 37,8 $cm^3$ (0,4 mole) de sulfate de méthyle. On porte pendant 2 heures au reflux, puis on ajoute 50 $cm^3$ d'eau et l'on chauffe pendant 2 heures. On évapore le THF, on reprend le résidu à l'éther, on lave à

l'eau, on sèche sur sulfate de sodium, on filtre et l'on évapore jusqu'à siccité. Les cristaux jaunes obtenus sont recristallisés dans l'hexane. On récupère 39,25 g de cristaux jaune d'or. Rendement: 83%.
Point de fusion: 95° C.
Chromatographie sur plaque:

- support: gel de silice 60 F 254 Merck
- solvant: acétate d'éthyle – éther de pétrole 30/70
- révélation: U.V. et iode
- Rf: 0,86.

(c) Préparation du N-méthyl benzoyl-2′ dibromo-2-4′ acétanilide

A une solution de 11,4 g (0,039 mole) de méthylamino-2 bromo-5 benzophénone dans 100 $cm^3$ d'acétate d'éthyle, on ajoute quelques glaçons puis 4,4 cc (0,05 mole) de bromure de bromacétyle et on agite pendant 3 heures sous forte agitation.

On décante, on lave à la soude (N) puis à l'eau jusqu'à neutralité, on sèche sur sulfate de sodium, on filtre, on évapore jusqu'à siccité. On obtient après trituration dans l'éther de pétrole 15 g de cristaux.
Rendement: 93%.
Point de fusion: 76° C.
Chromatographie sur plaque:

- support: gel de silice 60 F 254 Merck
- solvant: acétate d'éthyle – éther de pétrole 30/70
- révélation: U.V. et iode
- Rf: 0,5.

(d) Préparation du chlorhydrate de
N-cyclohexyl N′-méthyl benzoyl-2′ bromo-4′ glycylanilide

A une solution de 6,17 g de N-méthyl benzoyl-2′ dibromo-2-4′ acétanilide (0,015 mole) dans 50 $cm^3$ d'acétone, on ajoute 4,3 $cm^3$ (0,037 mole) de cyclohexylamine et on agite pendant 4 heures à température ambiante.

On évapore l'acétone, on traite par une solution de bicarbonate de sodium, on extrait à l'acétate d'éthyle, on décante, on lave à l'eau jusqu'à neutralité, on sèche sur sulfate de sodium, on filtre et on évapore jusqu'à siccité.

L'huile obtenue est dissoute dans une solution éthanolique d'acide chlorhydrique, et on précipite le chlorhydrate formé par addition d'éther isopropylique.

On récupère avec un rendement de 81% de produit de formule:

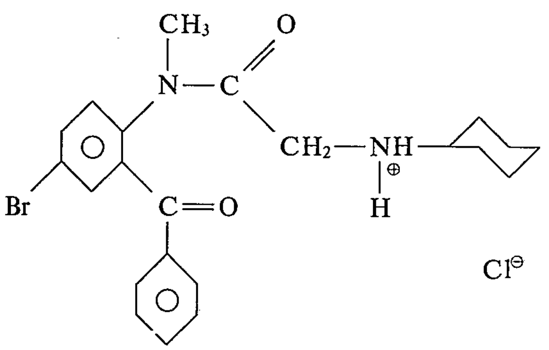

Formule brute: $C_{22}H_{26}Br\,Cl\,N_2O_2$
Masse moléculaire: 465,79
Cristaux blancs
Chromatographie sur plaque:
- support: gel de silice 60 F 254 Merck
- solvant: butanol – acide acétique – eau 6/2/2
- révélation: U.V. et iode
- Rf: 0,65

Exemple 5

N-méthyl benzoyl-2′ bromo-4′ morpholino-2 acétanilide

A une solution de 6,17 g (0,015 mole) de N-méthyl benzoyl-2′ dibromo-2-4′ acétanilide dans 50 $cm^3$ d'acétone, on ajoute 3,3 $cm^3$ (0,037 mole) de morpholine et on agite pendant 4 heures à la température ambiante.

On évapore l'acétone sous vide et on reprend le résidu par une solution de bicarbonate de sodium. On extrait à l'acétate d'éthyle, on lave à l'eau jusqu'à neutralité et on sèche sur sulfate de sodium.

On filtre et on évapore jusqu'à siccité; le résidu est recristallisé dans l'acétate d'éthyle-hexane. On récupère avec un rendement de 87% le produit de formule:

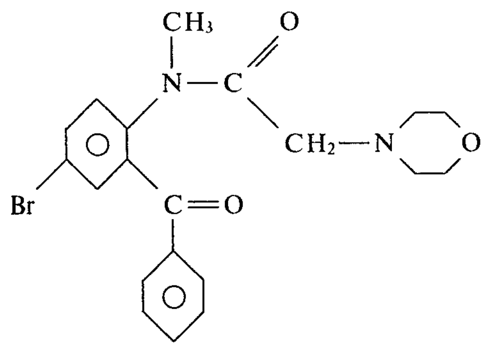

Formule brute: $C_{20}H_{21}Br\ N_2O_3$
Masse moléculaire: 417,3
Cristaux jaune clair
Point de fusion: 107° C
Chromatographie sur plaque:

- support: gel de silice 60 F 254 Merck
- solvant: méthanol – chloroforme 15/85
- révélation: U.V. et iode
- Rf: 0,63

Exemple 6

N-N-diméthyl N($\beta$ hydroxyéthyl) benzoyl-2′ bromo-4′ glycylanilide

A une solution de 6,17 g (0,015 mole) de N-méthyl benzoyl-2′ dibromo-2-4′ acétanilide dans 50 $cm^3$ d'acétone, on ajoute 3 $cm^3$ (0,037 mole) de N-méthyl éthanolamine et l'on agite pendant 4 heures à la température ambiante.

On évapore l'acétone, on reprend le résidu par une solution de bicarbonate et l'on extrait à l'acétate d'éthyle.

On décante, on lave à l'eau jusqu'à neutralité, on sèche sur sulfate de sodium, on filtre et on évapore jusqu'à siccité.

On récupère après recristallisation dans l'acétate d'éthyle-hexane 5,55 g de cristaux (rendement 91%) du produit de formule:

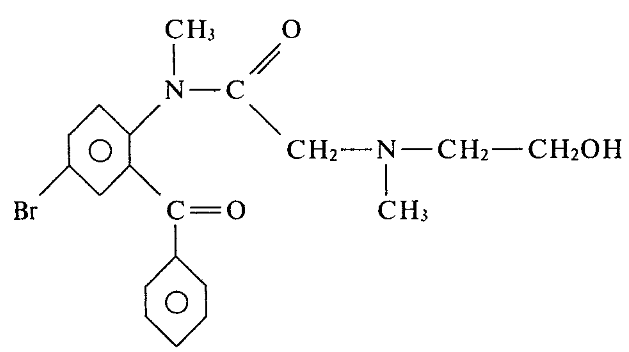

Formule brute: $C_{19}H_{21}Br\ N_2O_3$
Masse moléculaire: 405,29

Cristaux blancs
Point de fusion: 111° C
Chromatographie sur plaque:
- support: gel de silice 60 F 254 Merck
- solvant: méthanol – chloroforme 15/85
- révélation: U.V. et iode
- Rf: 0,41

Exemple 7

Chlorhydrate de N-N-bis (β hydroxy éthyl) N′ méthyl benzoyl-2′ bromo-4 glycylanilide

A une solution de 6,17 g de N-méthyl benzoyl-2′ dibromo-2-4′ acétanilide (0,015 mole) dans 50 $cm^3$ d'acétone, on ajoute 3,6 $cm^3$ (0,037 mole) de diéthanolamine et on chauffe pendant 3 heures à reflux.

On évapore l'acétone et on reprend le résidu par une solution bicarbonatée, on extrait à l'acétate d'éthyle, on décante et on lave à l'eau jusqu'à neutralité. On sèche sur sulfate de sodium, on filtre et on évapore le solvant jusqu'à siccite.

L'huile obtenue est traitée par une solution éthanolique d'acide chlorhydrique, par addition d'éther. On précipite le chlorhydrate que l'on recristallise dans un mélange isopropanol – méthanol. On récupère avec un rendement de 82% le produit de formule:

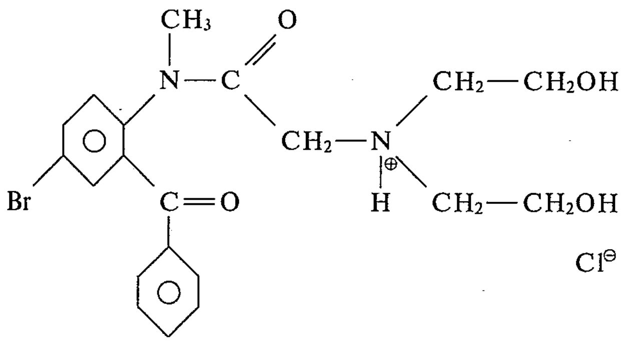

Formule brute: $C_{20}H_{24}Cl\ Br\ N_2O_4$
Masse moléculaire: 471,76
Cristaux blancs
Point de fusion: 179° C
Chromatographie sur plaque:
- support: gel de silice 60 F 254 Merck
- solvant: méthanol – chloroforme 15/85
- révélation: U.V. et iode
- Rf: 0,40

Exemple 8

Chlorhydrate de N-méthyl N′(méthyl-2′ allyl) benzoyl-2′ iodo-4′ glycylanilide

D'une manière analogue à celle décrite dans l'exemple 7, mais en condensant le N-méthyl benzoyl-2′ iodo-4′ bromo-2 acétanilide sur la méthyl-2 allylamine, on obtient le produit de formule:

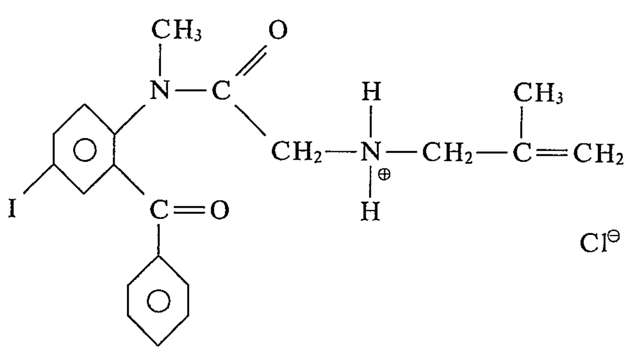

Exemple 9

N-méthyl N'-diméthyl-1,1 propargyl (benzoyl-2' méthyl-4') glycylanilide

D'une manière analogue à celle décrite dans l'exemple 7, mais en condensant le N-méthyl benzoyl-2' méthyl-4' bromo-2 acétanilide sur la diméthyl-1-1 propargylamine, on obtient le produit de formule:

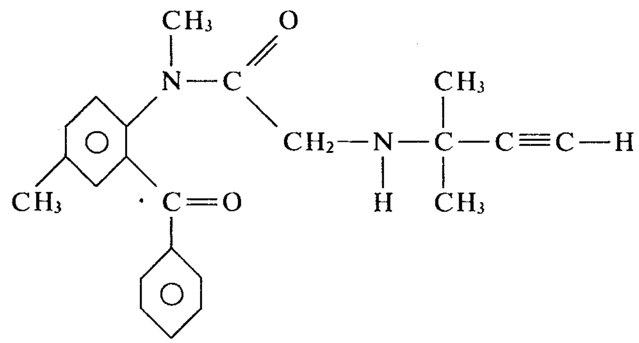

Exemple 10

Chlorhydrate de N-éthyl N' cyclopropyl (benzoyl-2' méthoxy-4') glycylanilide

D'une manière analogue à celle décrite dans l'exemple 7, mais en condensant le N-éthyl benzoyl-2' méthoxy-4' bromo-2 acétanilide sur la cyclopropylamine, on obtient le produit de formule:

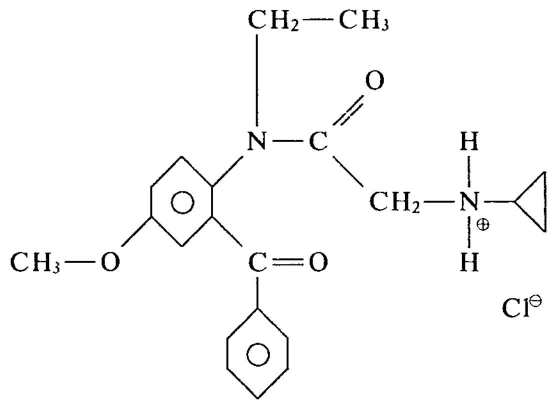

Exemple 11

N-méthyl N'-cyclohexyl (benzoyl-2' méthyl mercapto-4') glycylanilide

D'une manière analogue à celle décrite dans l'exemple 7, mais en condensant le N-méthyl benzoyl-2' méthylmercaptan-4' bromo-2 acétanilide sur la cyclohexylamine, on obtient le produit de formule:

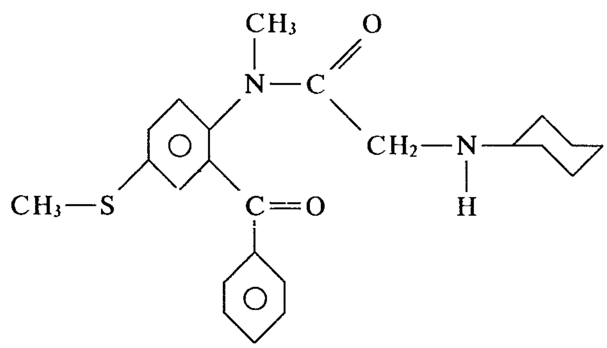

Exemple 12

N-méthyl N'-cyclohexyl (benzoyl-2' trifluorométhyl-4') glycylanilide

D'une manière analogue à celle décrite dans l'exemple 7, mais en condensant le N-méthyl benzoyl-2' trifluorométhyl-4' bromo-2 acétanilide sur la cyclohexylamine, on obtient le produit de formule:

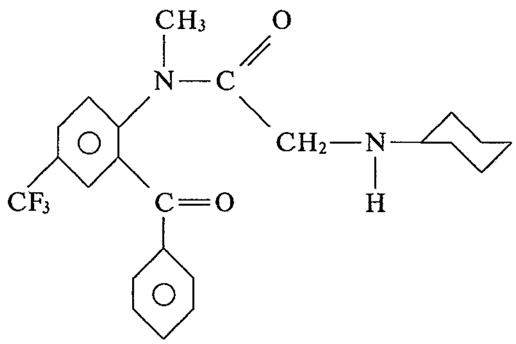

Exemple 13

Chlorhydrate de N-méthyl N'-cyclohexyl (benzoyl-2' cyano-4') glycylanilide

D'une manière analogue à celle décrite dans l'exemple 7, mais en condensant le N-méthyl benzoyl-2' cyano-4' bromo-2 acétanilide sur la cyclohexylamine, on obtient le produit de formule:

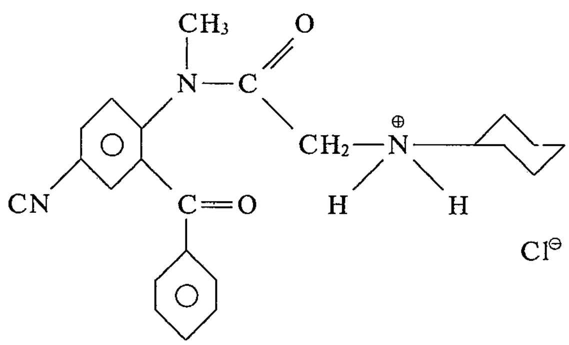

Exemple 14

N-méthyl N'-cyclohexyl (benzoyl-2' diméthylamino-4') glycylanilide

D'une manière analogue à celle décrite dans l'exemple 7, mais en condensant le N-méthyl benzoyl-2' diméthylamino-4' bromo-2 acétanilide sur la cyclohexylamine, on obtient le produit de formule:

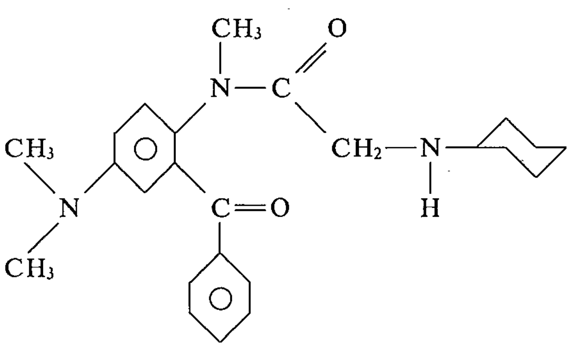

## Proprietes pharmacologiques

### 1) Etude de la toxicité

Les composés les plus actifs de la présente invention ont été soumis à des contrôles de toxicité.

La dose létale 50 et les activités pharmacologiques sont rapportées dans le Tableau (I).

La dose létale a été recherchée par voie orale et calculée selon la méthode de Miller et Tainter (Proc. Soc. Exper. Biol. Med., 1944, 57, 261).

### 2) Activité dans l'essai sur la tige tournante (Rota-Rod)

On réalise cet essai sur des souris mâles de souche swiss. On place la souris sur une tige de bois d'un diamètre de 3 cm, tournant à raison de cinq tours par minute. On choisit les souris qui peuvent rester sur la tige pendant au moins trois minutes au cours d'essais successifs et on les rassemble par groupe de dix pour l'essai de chaque dose.

Si la souris tombe de la tige en moins de deux minutes, on considère le composé essayé comme efficace.

Les résultats sont exprimés en $DE_{50}$ selon: N. W. Dunham et T. S. Miva (J. Amer. Pharm. Asso., 1957, 46, 208).

### 3) Activité antagoniste au pentétrazol

Ce test est réalisé sur un groupe de dix souris mâles de souche Swiss. Dans un délai de quinze minutes après l'injection sous-cutanée de 125 mg/kg de pentétrazol, les souris ont des convulsions toniques dont l'issue est fatale. Pour l'essai, on administre par voie orale les composés soixante minutes avant l'injection de pentétrazol. Les animaux sont observes pendant deux heures aprés administration du pentétrazol.

Les résultats sont exprimés par la dose efficace $DE_{50}$ selon Goodmann et Coll. (J. Pharmacol. 108, 1953).

Résultats (Tableau I)

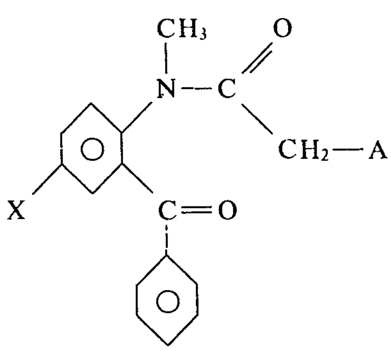

| X | A | Toxicité v. o. $DE_{50}$ mg/kg | Rota-Rod v. o. $DE_{50}$ mg/kg | Pentétrazol v. o. $DE_{50}$ mg/kg |
|---|---|---|---|---|
| Br | 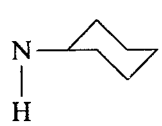 | >1000 | 22 | 4 |
| Br | 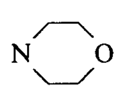 | >1000 | 20 | 3 |
| Br | 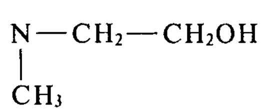 | >1000 | 18 | 1,5 |

Fortsetzung

| X | A | Toxicité v. o. $DE_{50}$ mg/kg | Rota-Rod v. o. $DE_{50}$ mg/kg | Pentétrazol v. o. $DE_{50}$ mg/kg |
|---|---|---|---|---|
| Br | 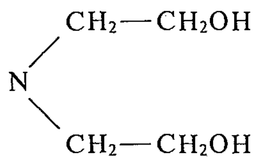 | >1000 | 15 | 1,3 |
| $CF_3$ | 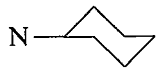 | >1000 | 8 | 1 |
| CN | 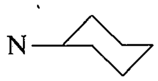 | >1000 | 7 | 0,9 |

Applications therapeutiques

Compte tenu de leurs propriëtés pharmacologiques et leur faible toxicité, ces composés chimiques peuvent être utilisés en thérapeutique dans le traitement de l'anxiété et des névroses.

Ces composés et leurs sels d'addition d'acides thérapeutiquement compatibles peuvent être utilisés comme médicaments, par exemple sous forme de préparations pharmaceutiques adaptées à l'administration entérale ou parentérale, avec par exemple, l'eau, le lactose, la gélatine, les amidons, le stéarate de magnésium, le talc, les huiles végétales, les gommes, les polyalcoylèneglycols, la vaseline, etc.

Ces préparations peuvent se présenter sous forme solide, par exemple de comprimés, dragées, capsules, etc. ou sous forme liquide, par exemple de solutions, suspensions ou émulsions.

Les préparations pharmaceutiques sous une forme appropriée à l'injection sont préférées. Ces préparations peuvent être soumises à des opérations pharmaceutiques classiques telles que la stérilisation et/ou peuvent contenir des adjuvants par exemple des agents conservateurs, stabilisants, de mouillage ou d'émulsification, des composés-tampons, etc.

Les dosages, auxquels les composés actifs et leurs sels d'addition d'acide thérapeutiquement compatibles peuvent être administrés, peuvent varier dans des proportions importantes selon l'état du patient. Un dosage quotidien d'environ 0,01 mg à 1 mg par kg de poids corporel est toutefois préfére.

Les compositions pharmaceutiques selon l'invention peuvent être utilisées en médecine interne, par exemple dans le traitement d'états pathologiques organiques, tels que l'hypertension artérielle et les coronarites, accompagnés et aggravés par un état anxieux; en médecine psychosomatique, par exemple pour le traitement de l'asthme, des ulcères gastroduodénaux, des colopahties et d'autres affections digestives fonctionnelles, ainsi qu'en psychiatrie, par exemple pour les traitements d'états d'agitation anxieux chez les sujets psychotiques.

Bien entendu, la présente invention ne se trouve pas limitée aux exemples particuliers mentionnés à simple titre illustratif, mais il est parfaitement possible, sans pour autant sortir du cadre de l'invention, d'en imaginer un certain nombre de variantes et de modifications. Ainsi, la présente invention s'étend également aux compositions pharmaceutiques contenant, outre les composés de formule I de la présente invention, d'autres principes actifs associés venant compléter ou renforcer l'action thérapeutique des composés de formule I.

**Revendications**

1. Nouveaux dérivés de benzoyl-2 glycylanilides substitués répondant à la formule générale I

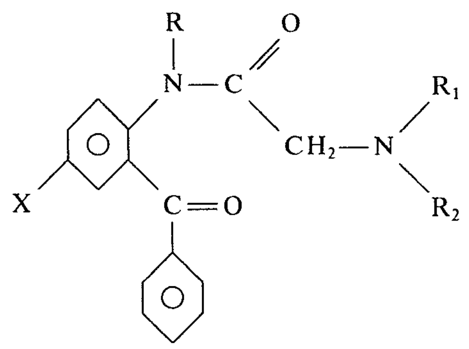

(I)

dans laquelle

X représente un atome d'hydrogène, de brome, de fluor ou d'iode, ou un groupe alcoyle inférieur, alcoxy inférieur, un groupe cyané, un groupe trifluorométhyle, un groupe alcoyl-mercapto ou un groupe dialcoylamino;

R représente un groupe alcoyle inférieur;

$R_1$ et $R_2$ peuvent être identiques ou différents et sont choisis parmi l'hydrogène, les groupes alcoyle inférieur, hydroxy-alcoyle inférieur, alcényle inférieur, alcynyle inférieur, un cycloalcoyle de 3 à 6 chaînons éventuellement substitués par un radical alcoyle inférieur; ils peuvent en outre former avec l'atome d'azote auquel ils sont reliés, un hétérocycle azoté comprenant éventuellement un second hétéroatome choisi parmi l'oxygène et l'azote;

à la condition que lorsque $R_1 = H$, $R_2$ est différent d'un alcoyle inférieur ou d'un groupe hydroxyalcoyle inférieur, et

$R_1$ et $R_2$ ne peuvent représenter simultanément un atome d'hydrogène ou un groupe alcoyle inférieur, ainsi que leurs sels obtenus avec des acides minéraux ou organiques thérapeutiquement acceptables.

2. Le N-méthyl benzoyl-2 morpholino-2 acétanilide de formule générale I selon la revendication 1.

3. Le N-N' diméthyl N(hydroxy-2 éthyl) benzoyl-2' glycylanilide de formule générale I selon la revendication 1.

4. Le maléate acide de N-cyclohexyl N'-méthyl (benzoyl-2' fluoro-4') glycylanilide de formule générale I selon la revendication 1.

5. Le chlorhydrate de N-cyclohexyl N'-methyl (benzoyl-2' bromo-4') glycylanilide de formule générale I selon la revendication 1.

6. Le N-méthyl benzoyl-2' bromo-4' morpholino-2 acétanilide de formule générale I selon la revendication 1.

7. Le N-N' diméthyl N($\beta$ hydroxy éthyl) benzoyl-2' bromo-4' glycylanilide de formule générale I selon la revendications 1.

8. Le chlorhydrate de N-N-bis ($\beta$ hydroxy éthyl) N'-méthyl benzoyl-2' bromo-4' glycylanilide de formule générale I selon la revendications 1.

9. Le chlorhydrate de N-méthyl N'(méthyl-2' allyl) benzoyl-2' iodo-4' glycylanilide de formule générale I selon la revendication 1.

10. Le N-méthyl N'-diméthyl-1-1 propargyl (benzoyl-2' méthyl-4') glycylanilide de formule générale I selon la revendication 1.

11. Le chlorhydrate de N-éthyl N'-cyclopropyl (benzoyl-2' méthoxy-4') glycylanilide de formule générale I selon la revendication 1.

12. Le N-méthyl N' cyclohexyl (benzoyl-2' méthyl mercapto-4') glycylanilide de formule générale I selon la revendication 1.

13. Le N-méthyl N' cyclohexyl (benzoyl-2' trifluorométhyl-4') glycylanilide de formule générale I selon la revendication 1.

14. Le chlorhydrate de N-méthyl N'-cyclohexyl (benzoyl-2' cyano-4') gycylanilide de formule générale I selon la revendication 1.

15. Le N-méthyl N'-cyclohexyl (benzoyl-2' diméthylamino-4') glycylanilide de formule générale I selon la revendication 1.

16. Procédé de préparation des composés de formule générale I selon l'une des revendications 1 à 15, caracterisé par le fait qu'il consiste à faire réagir un haloacétamide de formule générale (II)

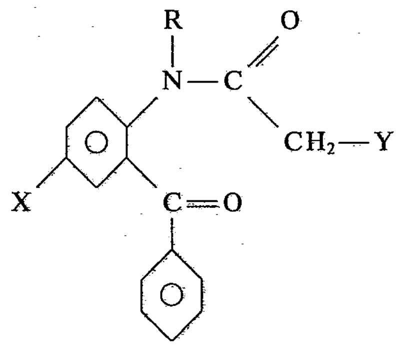

(II)

dans laquelle:

R et X ont les significations données à la revendication 1, et
Y représente un atome d'halogène,

avec une amine de formule générale (III):

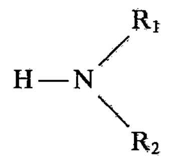

(III)

dans laquelle:

$R_1$ et $R_2$ ont les significations données à la revendication 1.

17. A titre de médicaments nouveaux utiles comme tranquillisants, anxiolytiques et hypnotiques, les composés selon l'une des revendications 1 à 15.

18. Les compositions pharmaceutiques contenant comme principes actifs au moins un composé selon l'une des revendications 1 à 15.

19. Les compositions pharmaceutiques selon la revendication 18 dans lesquelles aux produits selon l'une des revendications 1 à 15, peuvent être associés d'autres principes actifs venant compléter ou renforcer leurs actions thérapeutiques.

**Patentansprüche**

1. Neue substituierte 2-(Benzoyl)-glycylanilide der allgemeinen Formel

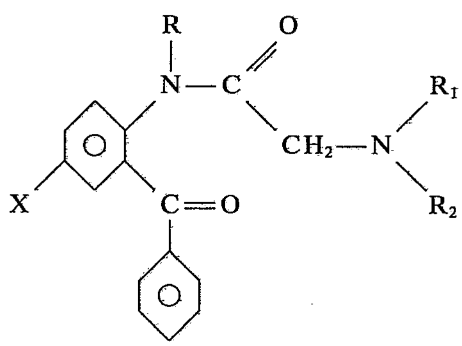

(I)

worin

X Wasserstoff, Brom, Fluor oder Jod oder eine niedere Alkylgruppe, eine niedere Alkoxygruppe, eine Cyanogruppe, ein Trifluormethylgruppe, eine Alkylmercaptogruppe oder eine Dialkylaminogruppe bedeutet,
R eine niedere Alkylgruppe darstellt und
$R_1$ und $R_2$, welche untereinander identisch oder voneinander verschieden sein können, für Wasserstoff, niedere Alkylgruppen, niedere Hydroxyalkylgruppen, niedere Alkenylgruppen, niedere Alkinylgruppen oder gegebenenfalls durch eine niedere Alkylgruppe substituierte

Cycloalkylgruppen mit 3 bis 6 Ringatomen stehen und zusammen mit dem diese Reste tragenden Stickstoffatom einen stickstoffhaltigen und gegebenenfalls ein von Sauerstoff oder Stickstoff gebildetes weiteres Heteroatom enthaltenden heterozyklischen Ring bilden können,

wobei, falls $R_1=H$, $R_2$ von einer niederen Alkylgruppe oder einer niederen Hydroxyalkylgruppe verschieden ist und
wobei $R_1$ und $R_2$ nicht gleichzeitig für Wasserstoff oder eine niedere Alkylgruppe stehen können, und ihre Salze mit therapeutisch verträglichen Mineralsäuren oder organischen Säuren.

2. N-Methyl-(2'-benzoyl)-2-morpholino-acetanilid der allgemeinen Formel (I) gemäß Anspruch 1.

3. N,N'-Dimethyl-N-(2-hydroxy-äthyl)-2'-benzoyl-glycylanilid der allgemeinen Formel (I) gemäß Anspruch 1.

4. Saures Maleat von N-Cyclohexyl-N'-methyl-(2'-benzoyl-4'-fluor)-glycylanilid der allgemeinen Formel (I) gemäß Anspruch 1.

5. Hydrochlorid von N-Cyclohexyl-N'-methyl-(2-benzoyl-4'-brom)-glycylanilid der allgemeinen Formel (I) gemäß Anspruch 1.

6. N-Methyl-2'-benzoyl-4'-brom-2-morpholino-2-acetalinid der allgemeinen Formel (I) gemäß Anspruch 1.

7. N,N'-Dimethyl-N-($\beta$-hydroxyäthyl)-2'-benzoyl-4'-brom-glycylanilid der allgemeinen Formel (I) gemäß Anspruch 1.

8. Hydrochlorid von N,N-Bis-($\beta$-hydroxyäthyl)-N'-methyl-2'-benzoyl-4'-brom-glycylanilid der allgemeinen Formel (I) gemäß Anspruch 1.

9. Hydrochlorid von N-Methyl-N'-(2-methyl-allyl)-2'-benzoyl-4'-jod-glycylanilid der allgemeinen Formel (I) bemäß Anspruch 1.

10. N-Methyl-N'-(1,1-dimethyl-propargyl)-2'-benzoyl-4'-methyl)-glycylanilid der allgemeinen Formel (I) gemäß Anspruch 1.

11. Hydrochlorid von N-Äthyl-N'-cyclopropyl-(2'-benzoyl-4'-methoxy)-glycylanilid der allgemeinen Formel (I) gemäß Anspruch 1.

12. N-Methyl-N'-cyclohexyl-(2'-benzoyl-4'-methyl-mercapto)-glycylanilid der allgemeinen Formel (I) gemäß Anspruch 1.

13. N-Methyl-N'-cyclohexyl-(2'-benzoyl-4'-trifluor-methyl)-glycylanilid der allgemeinen Formel (I) gemäß Anspruch 1.

14. Hydrochlorid von N-Methyl-N'-cyclohexyl-(2'-benzoyl-4'-cyano)-glycylanilid der allgemeinen Formel (I) gemäß Anspruch 1.

15. N-Methyl-N'-cyclohexyl-(2'-benzoyl-4'-dimethyl-amino)-glycylanilid der allgemeinen Formel (I) gemäß Anspruch 1.

16. Verfahren zum Herstellen von Verbindungen der allgemeinen Formel (I) gemäß einem der Ansprüche 1 bis 15, dadurch gekennzeichnet, daß ein Halogenacetamid der allgemeinen Formel

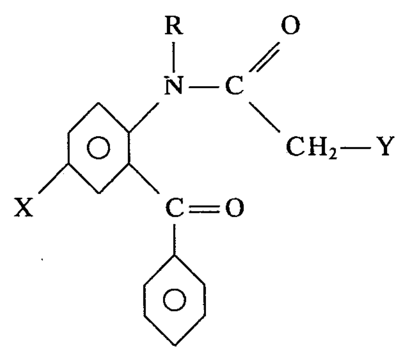

(II)

worin

R und X die im Anspruch 1 angegebene Bedeutung besitzen und
Y ein Halogen darstellt, mit einem Amin der allgemeinen Formel

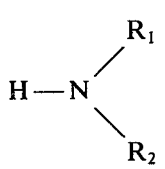

(III)

worin

$R_1$ und $R_2$ die im Anspruch 1 angegebene Bedeutung besitzen,

umgesetzt wird.

17. Verbindungen gemäß einem der Ansprüche 1 bis 10 als Tranquilisatoren, Anxiolytika und Hypnotika verwendbare neue Arzneimittel.

18. Pharmazeutische Mischungen, welche als Wirkstoff zumindest eine Verbindung gemäß einem der Ansprüche 1 bis 15 enthalten.

19. Pharmazeutische Mischungen nach Anspruch 18, in welchen den Verbindungen gemäß einem der Ansprüche 1 bis 15 deren therapeutische Wirkung ergänzende oder verstärkende weitere Wirkstoffe zugesetzt sind.

## Claims

1. New derivatives of substituted 2-benzoyl glycinanilides corresponding to the following general formula I:

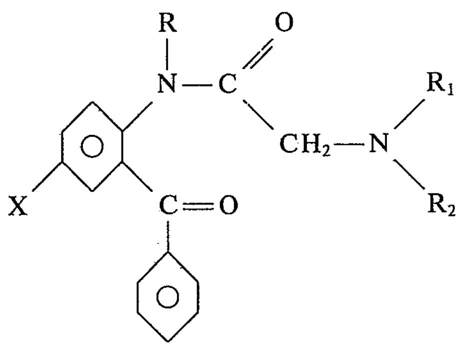

(I)

wherein

X represents a hydrogen, bromine, fluorine or iodine atom, or a lower alkyl group, lower alkoxy group, a cyano group, a trifluoromethyl group, an alkyl-mercapto group or a dialkylamino group;

R represents a lower alkyl group;

$R_1$ and $R_2$ may be the same or different and are selected from hydrogen, lower alkyl, lower hydroxyalkyl, lower alkenyl and lower alkynyl groups, a cycloalkyl having from 3 to 6 ring members optionally substituted by a lower alkyl radical; in addition, $R_1$ and $R_2$ may together with the nitrogen atom to which they are connected, from a nitrogen-containing heterocycle optionally including a second heteroatom selected from oxygen and nitrogen;

provided that when $R_1 = H$, $R_2$ is not a lower alkyl or a lower hydroxyalkyl group, and $R_1$ and $R_2$ cannot simultaneously represent a hydrogen atom or a lower alkyl group,

together with the salts thereof obtained with therapeutically acceptable inorganic or organic acids.

2. N-methyl-2-benzoyl-2-morpholino acetanilide of the general formula I according to claim 1.

3. N,N′-dimethyl-N(2-hydroxyethyl)-2′-benzoyl glycinanilide of the general formula I according to claim 1.

4. The acid maleate of N-cyclohexyl-N′-methyl (2′-benzoyl-4′-fluoro) glycinanilide of the general formula I according to claim 1.

5. The hydrochloride of N-cyclohexyl-N′-methyl (2′-benzoyl-4′-bromo) glycinanilide of the general formula I according to claim 1.

6. N-methyl-2′-benzoyl-4′-bromo-2-morpholino acetanilide of the general formula I according to claim 1.

7. N,N′-dimethyl-N($\beta$-hydroxyethyl) 2′-benzoyl-4′-bromo glycinanilide of the general formula I according to claim 1.

8. The hydrochloride of N,N-bis ($\beta$-hydroxyethyl) N′-methyl-2′-benzoyl-4′-bromo glycinanilide of the general formula I according to claim 1.

9. The hydrochloride of N-methyl-N′ (2′-methyl allyl)-2′-benzoyl-4′-iodo glycinanilide of the general formula I according to claim 1.

10. N-methyl N′-1,1-dimethylpropargyl-(2′-benzoyl-4′-methyl) glycinanilide of the general formula I according to claim 1.

11. The hydrochloride of N-ethyl-N′-cyclopropyl-(2′-benzoyl-4′-methoxy) glycinanilide of the general formula I according to claim 1.

12. N-methyl-N′-cyclohexyl-(2′-benzoyl-4′-methyl-mercapto) glycinanilide of the general formula I according to claim 1.

13. N-methyl-N′-cyclohexyl (2′-benzoyl 4′-trifluoromethyl) glycinanilide of the general formula I according to claim 1.

14. The hydrochloride of N-methyl-N'-cyclohexyl-(2'-benzoyl-4'-cyano) glycinanilide of the general formula I according to claim 1.

15. N-methyl-N'-cyclohexyl-(2'-benzoyl-4'-dimethylamino) glycinanilide of the general formula I according to claim 1.

16. A process for the preparation of compounds of the general formula I according to one of claims 1 to 15, characterised in that it consists of reacting a haloacetamide of the following general formula (II):

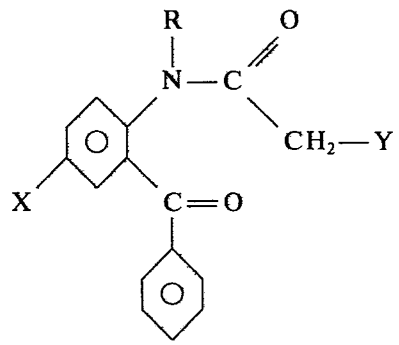

(II)

wherein:

R and X have the meanings given in claim 1, and

Y represents a halogen atom, with an amine of the following general formula (III):

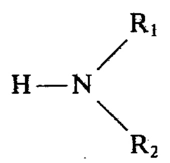

(III)

wherein:

$R_1$ and $R_2$ have the meanings given in claim 1.

17. As new medicaments which may be used as tranquillizing, anxiolytic and hypnotic medicaments, the compounds according to one of claims 1 to 15.

18. Pharmaceutical compositions containing at least one compound according to one of claims 1 to 15 as active principles.

19. Pharmaceutical composions according to claim 18 in which other active principles may be associated with the products according to one of claims 1 to 15, to complete or strengthen their therapeutic actions.